(19) Europäisches Patentamt
European Patent Office
Office européen des brevets

(11) Veröffentlichungsnummer : **0 156 968**
**B1**

(12) # EUROPÄISCHE PATENTSCHRIFT

(45) Veröffentlichungstag der Patentschrift : **09.11.88**

(21) Anmeldenummer : **84114496.7**

(22) Anmeldetag : **29.11.84**

(51) Int. Cl.[4] : **C 07 F 7/18**, A 61 K 47/00, A 61 K 7/48

(54) **Umsetzungsprodukte von Fettsäure-polyol-partialestern und gamma-Glycidyl-oxipropyl-trialkoxysilanen, Verfahren zu deren Herstellung und deren Verwendung in pharmazeutischen und kosmetischen Präparaten.**

(30) Priorität : **23.12.83 DE 3346641**
**23.12.83 DE 3346642**

(43) Veröffentlichungstag der Anmeldung : **09.10.85 Patentblatt 85/41**

(45) Bekanntmachung des Hinweises auf die Patenterteilung : **09.11.88 Patentblatt 88/45**

(84) Benannte Vertragsstaaten :
**AT BE CH DE FR GB IT LI LU NL SE**

(56) Entgegenhaltungen :
**DE-B- 1 014 712**
**DE-B- 1 046 259**
**US-A- 3 258 477**

(73) Patentinhaber : **Hüls Troisdorf Aktiengesellschaft**
**Postfach 11 65**
**D-5210 Troisdorf (DE)**

(72) Erfinder : **Hülsmann, Hans Leo, Dr.**
**Appendahl 29**
**D-5802 Wetter 4 (Ruhr) (DE)**
Erfinder : **Pass, Reinhard**
**Brink 10**
**D-5810 Witten (DE)**
Erfinder : **Hermsdorf, Horst, Dr.**
**Vormholzstrasse 14 e**
**D-4630 Bochum-Querenburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft viskose gelartige Estergemische und deren Herstellung.

Ziel der Erfindung ist es, viskose gelartige Estergemische aus einfach zugänglichen Ausgangsstoffen nach wenig aufwendigen Verfahren herzustellen. Die Verwendung der viskosen gelartigen Substanzen erfolgt im technischen Sektor unter anderem zur Additivierung schmierungstechnischer Formulierungen sowie besonders im kosmetischen Bereich, in dem bevorzugt ihr Einsatz als viskose Ölkomponente oder Oleogel sowie als pharmazeutische Hilfsstoffe vorgesehen ist.

Bekannte viskose gelartige Estergemische auf Basis verschiedener Fettsäure-polyol-partialester sind nur begrenzt verwendbar. Die EP-B-0 014 308 beschreibt viskose Estergemische aus mit Dicarbonsäuren kondensierten Fettsäure-polyol-partialestern, deren Viskositätseinstellung jedoch durch den Übergang zu thermoplastischen Polyestern bei steigendem Dicarbonsäureeinbau begrenzt ist. Weiterhin können aus ungesättigten Ölen mit trockenen Eigenschaften durch thermische bzw. oxidative Polymerisation viskose gelartige Verbindungen hergestellt werden. Die Gelierung von Ölen, Fetten und darauf basierenden Partialestern in Zwei- oder Mehrkomponentensystemen zu Oleogelen mit Verdickungsmitteln, z. B. auf Seifenbasis, natürlichen Tonmineralien wie Bentonen, Siliziumoxid oder -hydroxid, Celluloseetherestern, Polyharnstoffverbunden oder Ureido- bzw. Amidoverbindungen ist bekannt. Häufig auftretende Mängel dieser ölgelbildenden Mehrstoffsysteme sind fehlende Transparenz, unangenehmes Hautgefühl, zu hohe Gelierungstemperaturen, beschränkte Möglichkeiten zur Einstellung niedrigviskoser Gelsysteme und besonders die Ausölung aus derartigen Gelen in Wirkstoffzubereitungen.

Es bestand daher die Aufgabe, viskose gelartige Esterbemische mit klarer Transparenz und hoher Beständigkeit der Gele herzustellen, deren Viskosität nach Möglichkeit auf vorher bestimmte Werte einstellbar ist.

Gegenstand der Erfindung sind viskose gelartige Estergemische nach Patentanspruch 1, bestehend aus Umsetzungsprodukten von Fettsäure-polyol-partialestern oder deren Gemischen mit Hydroxylzahlen von 5 bis 150, vorzugsweise 10 bis 80 und 2 bis 45 Gew.-%, vorzugsweise 4 bis 30 Gew.-% bezogen auf die Estergemische von $\gamma$-Glycidyl-oxipropyl-trialkoxisilanen mit Alkylgruppen von 1 bis 3-C-Atomen oder deren Gemischen sowie Estergemische nach Patentanspruch 2, welche als weitere Reaktionskomponente Dicarbonsäuren bzw. deren Anhydride bzw. Halogenide in Mengen von 0,01 bis 50 Mol-%, vorzugsweise 0,01 bis 30 Mol-%, je Mol, OH-Gruppen der Polyole enthalten.

Mit steigenden Hydroxylzahlen von 5 bis 150 der umgesetzten Partialester werden bei entsprechend steigenden Gehalten von 2 bis 45 Gew.-% $\gamma$-Glycidyl-oxipropyl-trialkoxisilanen Estergemische von steigender Viskosität erhalten.

Soweit Dicarbonsäuren als Reaktionskomponente enthalten sind, bewirkt deren steigender Gehalt eine geringere Steigerung der Viskosität, jedoch vor allem niedrige Trübungspunkte.

Die Viskositäten der erfindungsgemäßen Estergemische sind demnach durch Wahl der Komponenten und deren verwendeten Anteilen variiierbar und reproduzierbar.

Der Begriff Fettsäure-polyol-partialester bezeichnet die Stoffklasse der Hydroxylgruppen enthaltenden Fettsäureester von di- bis hexafunktionellen Alkoholen und deren Etherpolyolen. Mehrwertige Alkohole, d. h. Polyole sind beispielsweise Propandiol, Glycerin, Trimethylolpropan, Pentaerythrit oder Sorbit. Das Spektrum der zu Partialestern veresterbaren Säuren umfaßt gesättigte, ungesättigte, gerad- und verzweigtkettige, aliphatische und cycloaliphatische Monocarbonsäuren mit 2 bis 22 C-Atomen oder deren Gemische sowie ggfls. zusätzlich veresterbare gesättigte, ungesättigte, aliphatische und/oder aromatische Dicarbonsäuren, vorzugsweise aliphatische Dicarbonsäuren mit 2 bis 20, besonders 3 bis 8 C-Atomen. Die für den kosmetischen Bereich in Frage kommenden viskosen gelartigen Estergemische enthalten besonders Polyole wie Propandiol-1,2, Mono-, Di- und Triäthylenglykolen oder Glycerin und besonders Fettsäuren aliphatischer, geradkettiger oder verzweigtkettiger, insbesondere gesättigter Art, mit 6 bis 18 C-Atomen. Zusätzlich können besonders in die partielle Veresterung Dicarbonsäuren wie Bernsteinsäure, Adipin- oder Sebacinsäure mit einbezogen werden.

Viskose gelartige Estergemische, die im technischen Sektor Verwendung finden, können Polyole wie z. B. Trimethylolpropan, Pentaerythrit und verzweigtkettige und/oder geradkettige Fettsäuren mit 6 bis 18 C-Atomen enthalten. Die partielle Mitveresterung von Dicarbonsäuren ist möglich.

Die umzusetzenden Fettsäure-polyol-partialester enthalten stets freie Hydroxylgruppen der verwendeten Polyole, deren Menge durch die Hydroxylzahl (OHZ, gemessen in mg KOH je g Ester) ausgedrückt ist.

Die umgesetzten Silane gehen durch Reaktion der Epoxigruppe des Glycidylrests mit diesen Hydroxygruppen der Partialester eine Bindung ein.

Im Falle von Partialestern mit Gehalten von Dicarbonsäuren besitzen diese ebenfalls die Hydroxylzahlen im genannten Bereich. Die Säurezahl (SZ) ist dann bevorzugt O oder sehr klein. Soweit durch Art der Reaktionsführung die zweite Carboxylgruppe der Dicarbonsäuren nicht gebunden ist, kann ggfls. eine geringe oder größere Säurezahl, zusätzlich zur Hydroxylzahl, auftreten.

Neutrale Ester oder Vollester der Polyole mit den genannten Säuren besitzen demgegenüber Hydroxylzahlen und Säurezahlen von O oder sehr kleinen Werten. Soweit durch Reaktion von Partialestern mit einem molaren Überschuß von Dicarbonsäuren Ester gebildet werden haben diese die

OHZ O und eine durch freie Carboxylgruppen bedingte Säurezahl.

Die Herstellung derartiger Partialester mit definierten Hydroxylgruppengehalten kann durch bekannte katalystische Veresterung der Polyolkomponenten mit einer gegenüber der stöchiometrischen Menge verminderten Menge Fettsäure oder durch Reaktion von Vollestern der Fettsäuren bzw. Estern der Fettsäuren und Dicarbonsäure einer Umesterung nach der EP 0 014 308 mit Polyolen in Art einer Umesterung in Gegenwart von Alkali oder Säuren erfolgen.

Die benötigten Organo-organosilane sind bekannt (Vgl US-A-3 258 477) und stehen aus der Produktion der Firma DYNAMIT NOBEL AG zur Verfügung. Bevorzugt sind $\gamma$-Glycidyl-oxipropyl-trimethoxisilan, $\gamma$-Glycidyl-oxipropyl-tripropoxisilan oder deren Gemische. Ansich sind Silane mit einer Epoxigruppe in einem Rest beliebiger Art sowie ein bis drei Alkoxigruppen beliebiger Art sowie einer ergänzenden Zahl inerter Reste verwendbar, jedoch sind solche Silane schwer zugängig.

Die viskosen gelartigen Estergemische sind im allgemeinen Neutralester ohne oder mit geringen Hydroxyl- und Säurezahlen.

Für besondere Zwecke können durch Wahl der Herstellbedingungen und Mengen der Komponenten Partialester mit OHZ von 5 bis zu 150 oder Carboxylgruppen enthaltende Ester mit SZ bis zu 150 erhalten werden.

Die erfindungsgemäßen Estergemische zeichnen sich durch folgende besondere Vorteile aus :

1. Es sind geruchlose, einheitliche, viskose, gelartige Estergemische von klarer Transparenz und angenehmen Hautgefühl.

2. Die Stabilität der viskosen gelartigen Estergemische ist ausgezeichnet, da die Gelgerüstbildung durch Verknüpfung über Hauptvalenzen erfolgt.

3. Die erhaltenen viskosen gelartigen Estergemische zeigen keine Neigung zur Ausölung bei Verwendung in pharmazeutischen und kosmetischen Zubereitungen oder zur Additivierung technischer Formulierungen. Selbst Phasenumwandlungen fest-flüssig sind reversibel.

4. Sie sind in Mineralölen paraffinischer und aromatischer Art sowie in Esterölen weitgehend löslich bzw. dispergierbar und können zu deren Viskositätseinstellung verwendet werden.

5. Es lassen sich viskose gelartige Estergemische mit Tropfpunkten von oberhalb 300 °C herstellen.

6. Die Verdampfungsverluste bei erhöhten Temperaturen werden durch den Einbau der Silanverbindung minimiert.

7. Die viskosen gelartigen Estergemische weisen hervorragende gerüstbildende Eigenschaften in Cremes und Lotionen auf

8. Die Viskosität bzw. Gelbeschaffenheit der erhaltenen Estergemische kann beliebig, je nach Einbau der Organo-organo-oxi-silane in die Fettsäure-polyol-partialester eingestellt werden.

Die Silanreste sind bevorzugt gänzlich durch Abspaltung von Alkohol zu Siloxangruppe Si-O-Si kondensiert, jedoch können ggfls. auch die Alkoxigruppen teilweise, in Ausnahmefällen auch ganz erhalten sein.

Die Herstellung der viskosen gelartigen Estergemische kann im allgemeinsten Fall durch Reaktion der Silankomponente mit Polyolestern erfolgen, welche vorzugsweise Hydroxylgruppen entsprechend den Hydroxylzahlen 5 bis 150 aufweisen und Monocarbonsäurereste sowie ggfls. Dicarbonsäurereste aufweisen oder ggfls. mit Polyolestern, welche Carboxylgruppen in entsprechender Menge mit Säurezahlen von 5 bis 150 aufweisen durch Gehalte von Dicarbonsäuren. Auch die Umsetzung von Partialestern mit einem stöchiometrischen Unterschuß der Silankomponente ist möglich.

Die Partialester können auf beliebige Weise vor der Reaktion hergestellt werden, ebenso Ester mit Gehalten von Carboxylgruppen, wobei bekannte Veresterungskatalysatoren, besonders Titanester verwendet werden.

Die Umsetzung mit den Silanen erfolgt bevorzugt in Anwesenheit von Katalysatoren, z. B. Lewis-Säuren bekannter Art, Toluolsulfonsäure oder $AlCl_3$ oder $SnCl_4$.

Die gleichen Katalysatoren sind bei der siloxanartigen Kondensation geeignet. Geeignete Katalysatoren sind in Ullmann Band 21 S. 515 angegeben. Soweit Alkoxigruppen erhalten bleiben sollen wird kein oder nur ein Teil des Alkohols abgespalten bzw. die Temperatur niedrig auf, z. B. 120 bis 140 °C gehalten. Zweckmäßig ist verminderter Druck von 2 bis 1 000 mbar, besonders 100 bis 200 mbar.

Fettsäure-polyol-partialester, die schwer zugängig sind, sowie Ester mit Gehalten von Carboxylgruppen können direkt vor der Umsetzung mit dem Silan, z. B. aus Neutralestern oder aus den Komponenten unter an sich bekannten Bedingungen der Umesterung bzw. Veresterung hergestellt werden, wobei Herstellung im selben Gefäß möglich ist.

Soweit eine Dicarbonsäure-komponente enthalten ist, kann diese durch Zusatz der Dicarbonsäure oder deren Derivaten, besonders den Anhydridengemischen, Anhydriden oder Halogeniden, besonders Chloriden erhalten werden.

Weiterer Gegenstand der Erfindung ist daher ein Verfahren zur Herstellung viskoser gelartiger Estergemische nach Anspruch 3, dadurch gekennzeichnet, daß man Fettsäurepolyol-partialester oder deren Gemische mit Hydroxylzahlen von 5 bis 150, bevorzugt 10 bis 80 mit 2 bis 45 Gew.-%, vorzugsweise 4 bis 30 Gew.-%, bezogen auf die Estergemische von $\gamma$-Glycidyl-oxipropyl-trialkoxisilane mit Alkylgruppen von 1 bis 3 C-Atomen bei 20 bis 210 °C, vorzugsweise 60 bis 120 °C, umsetzt und die freien Alkoxigruppen der Silane bei 100 bis 240 °C, vorzugsweise 160 bis 200 °C, unter Wasserdampfdurchleitung bei Dampftemperaturen von 100 bis 240 °C, bevorzugt 150 bis 170 °C, zu viskosen gelartigen Estergemischen ganz oder teilweise siloxanartig kondensiert oder Fettsäure-polyol-partialester mit Hydroxylzahlen von 5 bis 150 durch Umsetzung von Fettsäure-polyol-vollestern durch Umsetzung mit Polyolen bildet und darauf mit 2 bis 45 Gew.-% -Glycidyl-oxipropyl-trialkoxysilanen umsetzt und entsprechend siloxanartig kondensiert sowie ein Verfahren nach Anspruch 4, dadurch gekennzeichnet, daß man Dicarbonsäuren oder deren Anhydride bzw. Halogenide in Mengen von 0,01 bis 50 Mol-% OH-Gruppen der Polyole mit Fettsäure-polyol-partialestern zu solchen der Hydroxylzahlen von 5 bis 150 vor der Reaktion mit γ-Glycidyl-oxipropyl-trialkoxysilanen mit Dicarbonsäuren oder deren Derivaten umsetzt und die Verwendung der viskosen gelartigen Estergemische als Gelbildner oder Hilfsstoffe in pharmazeutischen oder kosmetischen Präparaten.

Die Erfindung betrifft weiterhin die Verwendung der silanmodifizierten Estergemische als Gelbildner oder Hilfsstoffe in pharmazeutischen oder kosmetischen Präparaten.

Aufgrund der viskosen gelartigen Konsistenz der Estergemische besteht eine besondere Eignung als Gelbildner bzw. als die Viskosität steigerndes Mittel in öligen Zubereitungen oder in Salbenzubereitungen bzw. zur Herstellung von Gelen. Mengenzusätze von 2 bis 80, vorzugsweise von 5 bis 40 % und sehr bevorzugt von 5 bis 15 % sind, je nach Viskosität der Estergemische, ausreichend.

Die Erfindung betrifft andererseits streichfähige Zubereitungen mit hoher Haftfestigkeit auf der Haut auf Basis der viskosen gelartigen silanmodifizierten Estergemische für die Verwendung als pharmazeutische oder kosmetische Präparate.

Die Zubereitung enthält eine Komponente von öliger Konsistenz sowie ggf. einen Wirkstoff bzw. Wirkstoffgehalte in der Komponente von öliger Konsistenz sowie das neue viskose gelartige Estergemisch, welches neue bisher nicht bekannte Eigenschaften der Zubereitung bewirkt.

Streichfähig bedeutet im Zusammenhang der Erfindung die Fähigkeit, die menschliche oder tierische Haut sowohl der Hautoberfläche wie auch der oberflächennahen Schleimhäute und der Schleimhäute im Körperinneren zu bedecken und sich auf diesen zu einem gleichmäßigen Film auszubreiten. Dementsprechend betrifft die Erfindung Zubereitungsformen aller hierfür geeigneten Konsistenzen von leichtflüssigen Zubereitungen, insbesondere leichtflüssigen Ölen von geringer Viskosität bis zu Crems, Salben, Emulsionen sowie insbesondere Gelen, welche die Beschaffenheit von fließfähigen Ölen bis zu steifen und stichfesten Gelen aufweisen können.

Seit langer Zeit bestand Bedarf für auf die Haut auftragbare Zubereitungen, welche eine bessere Haftung auf der Haut aufweisen, sich schwer von der Haut entfernen lassen und dadurch eine längere Wirkungszeit aufweisen bzw. eine längere Schutzwirkung der Haut gestatten.

Zubereitungen dieser Art standen jedoch nicht zur Verfügung, vielmehr sind die bekannten Zubereitungen häufig, zumindest mehrmals täglich erneut aufzubringen.

Wesentlicher Bestandteil der neuen streichfähigen Zubereitungen ist ein neues viskoses gelartiges Estergemisch, das aus einem Umsetzungsprodukt von Fettsäure-polyol-partialestern mit Hydroxylzahlen von 5 bis 150 mit γ-Glycidyl-oxipropyl-trialkoxisilanen besteht. Ggf. kann als Reaktionskomponente des Estergemisches auch der Rest einer Dicarbonsäure, insbesondere einer aliphatischen Dicarbonsäure, enthalten sein, in welchem Falle dann bevorzugt die Dicarbonsäure-Komponente einen Teil des genannten Partialesters der Hydroxylzahlen 5 bis 150 bildet.

Die weiteren Bestandteile der streichfähigen Zubereitung brauchen lediglich die Bedingung der Verträglichkeit im Rahmen von pharmazeutischen oder kosmetischen Zubereitungen zu erfüllen und von ölartiger Konsistenz zu sein.

In der ölartigen Zubereitung können weiterhin pharmazeutische oder kosmetische Wirkstoffe gelöst oder verteilt enthalten sein, wobei sie gesondert zugesetzt werden können oder bereits Bestandteil der Komponente von öliger Konsistenz sind.

Die Zubereitungen weisen jedoch in bestimmten Fällen keine zugesetzten oder in der öligen Komponente enthaltenen pharmazeutischen oder kosmetischen Wirkstoffe auf, da bereits die viskosen gelartigen Estergemische bestimmte neuartige pharmazeutische und kosmetische Wirkungen besitzen.

Es wurde nämlich gefunden, daß die genannten viskosen gelartigen Estergemische, welche unten eingehend beschrieben sind, eine extrem gute Haftung auf der Haut, den Schleimhäuten und menschlichem und tierischem Körpergewebe aller Art aufweisen. Diese besonders gute Haftung bewirkt insbesondere, daß die Zubereitungen schwer von der Haut entfernbar sind. Das behandelte Hautareal wird schwerbenetzbar und weist bei zahlreichen Zubereitungen einen milden oder auch starken Glanz auf. Durch übliche Wäsche mit Seife oder Waschmitteln sind die Zubereitungen zunächst nicht entfernbar, jedoch ist die Entfernung des Gels, je nach Gehalten des viskosen gelartigen Estergemisches und seiner Art, durch mehrmaliges Waschen mit z. B. der üblichen Seife möglich. Es sind Gele und andere Zubereitungen aus den Estergemischen herstellbar, welche erst durch 5- bis 6 maliges oder sogar erst durch 10 maliges Waschen entfernbar sind, so daß die Entfernung durch Waschen mit Seife in üblicher Art als Prüfung für die Haftfähigkeit benutzt wird.

Andererseits sind die viskosen gelartigen Estergemische und damit die streichfähigen Zubereitungen nach längerer Zeit durch die Haut völlig und ohne Nebenwirkungen resorbierbar, wobei ggf. vorhandene Wirkstoffe oder die Komponente von öliger Resistenz entsprechend ihrer eigenen Penetrationsfähigkeit zu einem früheren Zeitpunkt von der Haut resorbiert werden.

Die neuen viskosen gelartigen Estergemische haben weiterhin die bemerkenswerte Eigenschaft, besonders stabile Gelbildner zu sein, so daß Anteile der viskosen gelartigen Estergemische zur Einstellung der Viskosität der Komponente von ölartiger Konsistenz auf beliebige Werte von leichtflüssiger Konsistenz bis zu einem steifen Gel möglich ist. Durch die dreidimensionale Vernetzung mit Hilfe der Silankomponente im Umsetzungsprodukt der Estergemische entsteht eine dreidimensionale Gerüststruktur, die bereits bei kleinen Zusätzen Gele entstehen läßt, die die einmal eingestellte Viskosität auf sehr lange Zeiträume behalten und welche insbesondere auch bei wesentlich erhöhter Temperatur unter z. B. Tropenbedingungen die einmal eingestellte Viskosität beibehalten. Teilweise liegen die Tropfpunkte der viskosen gelartigen Estergemische höher als 300 °C. Weiterhin sind diese viskosen gelartigen Estergemische ausgezeichnete Emulgatoren, und zwar sowohl zur Herstellung von W/O- wie auch zur Herstellung von O/W-Emulsionen und ermöglichen damit die Herstellung von Salben, Crems, Lotionen u. dgl. Weiterhin besitzen die viskosen gelartigen Estergemische eine gute Spreitwirkung auf der Haut, wodurch einer angegriffenen oder durch häufiges Waschen geschädigten Haut die eigenen Fette erhalten bleiben bzw. eine rückfettende Wirkung aus z. B. pflegenden Hautcrems auftritt.

Insbesondere ist Gegenstand der Erfindung eine streichfähige Zubereitung mit hoher Haftfähigkeit auf der Haut, bestehend aus

a) 5 bis 80 Gew.-% viskose gelartige Estergemische, bestehend aus Umsetzungsprodukten von Fettsäure-polyol-partialestern oder deren Gemischen mit Hydroxylzahlen von 5 bis 150, vorzugsweise 10 bis 80, mit 2 bis 45 Gew.-%, vorzugsweise 4 bis 30 Gew.-%, bezogen auf die Estergemische, von $\gamma$-Glycidyloxipropyl-trialkoxisilanen mit Alkylgruppen von 1 bis 3 C-Atomen oder deren Gemischen oder ggf. bestehend aus Umsetzungsprodukten, die als weitere Reaktionskomponente Dicarbonsäuren bzw. deren Anhydride bzw. Halogenide in Mengen von 0,01 bis 50 Mol-%, vorzugsweise 0,01 bis 30 Mol-%, je Mol OH-Gruppen der Polyole enthalten

b) 95 bis 20 Gew.-% einer Komponente von öliger Konsistenz oder einer W/O- bzw. O/W-Emulsion aus diesem und Wasser sowie ggf.

c) 0,01 bis 20 Gew.-% eines Wirkstoffs oder dessen flüssiger oder ggf. fester Zubereitung.

Wie bereits gesagt, ist lediglich die Streichfähigkeit der Zubereitung und ein Gehalt der viskosen gelartigen Estergemische notwendige Voraussetzung für die Zubereitung gemäß der Erfindung, während die Gehalte des viskosen gelartigen Estergemisches auch Anteile unter 5 Gew.-% oder über 80 Gew.-% aufweisen können und die weitere Komponenten freibleibend sind, solange eine streichfähige Zubereitung für die Verwendung in pharmazeutischen und kosmetischen Zubereitungen entsteht.

Weiterer Gegenstand ist daher die Verwendung der Zubereitungen als pharmazeutische oder kosmetische Zubereitung, insbesondere zur Hautbehandlung.

Eine Verwendung der Zubereitung als Spray-Zubereitung mit Gehalten üblicher Verdünnungsmittel und Treibmittel ist möglich.

Die Zubereitungen können die Zubereitungsart von Ölen, Crems, Salben, Emulsionen oder Gelen von leichtstreichfähiger Konsistenz bis zu stichfesten Gelen haben, wobei insbesondere klare Gele von durchsichtiger bis durchscheinender Art herstellbar sind, sofern die weiteren Komponenten klar sind.

Die Zubereitungen besitzen, anders als zahlreiche Gele oder Salben, ein angenehmes Gefühl auf der Haut, welches durch die viskosen gelartigen Estergemische beigetragen wird.

Wie bereits gesagt, vermitteln die viskosen gelartigen Estergemische als Bestandteil der Zubereitungen eine extrem gute Haftung der Zubereitungen auf der Haut und ermöglichen bei hohen Anteilen der viskosen gelartigen Estergemische eine Erneuerung der Behandlung in langen Zeitabschnitten, jedoch ist bei geringen Gehalten der viskosen gelartigen Estergemische auch erreichbar, daß das Behandlungsmittel auf der Haut nicht mehr spürbar ist.

Wegen des guten Gelbildungsvermögens der Estergemische sind Zubereitungen als Gele, besonders als klare Gele, mit beliebigen weiteren Bestandteilen und ggf. Wirkstoffen ein besonderer Gegenstand der Erfindung. Nicht gelartige und nicht klare bzw. transparente Zubereitungen können besonders unter Nutzung der rückfettenden Eigenschaften und der Emulsionseigenschaften der Estergemische Anforderungen nach weiteren streichfähigen Zubereitungsarten, jedoch ebenfalls mit Vorteil hergestellt werden.

Ein weiterer besonderer Gegenstand der Erfindung sind Sonnenschutz- bzw. UV-Schutz-Zubereitungen unter Nutzung der besonders guten Haftung auf der Haut und der Beständigkeit bei Waschen der Haut mit Seife in üblicher Weise. Diese Sonnenschutzmittel mit Gehalten von bekannten Lichtschutzsubstanzen sind ebenso für den Sonnenschutz für sportliche Betätigung, beispielsweise für Wanderer und Skifahrer im Hochgebirge, Sonnenschutz während der sommerlichen Urlaubszeit wie auch für gewerbliche Zwecke, beispielsweise für den UV-Strahlenschutz von Elektroschweißern oder für Heilbehandlung unter Einwirkung von UV-Strahlen zum Hautschutz geeignet. Der Besondere Vorteil besteht darin, daß durch die besonders gute Haftung der enthaltenen Esterzubereitungen die durch die Sonnenschutzmittel behandelten Hautteile in Berührung mit Wasser, beispielsweise während eines Badeurlaubs, nicht benetzbar sind und so die übliche sehr häufige Benutzung der Sonnenschutzmittel durch die einfachere Auftragung in längeren Zeitabständen ersetzt werden kann.

Einen besonderen Wert haben die Zubereitungen auch als Hautschutzmittel, beispielsweise im gewerblichen Bereich zum Schutz vor aggressiven wäßrigen Medien und gleichermaßen zum Hautschutz im häuslichen Bereich als Bestandteil von tensid-haltigen Spülmitteln im Haushalt.

Gelzubereitungen, besonders klarer Gele, sind in vielen Fällen als Sonnenschutzmittel und Hautschutzmittel bevorzugt.

Beispiele weiterer Zubereitungen sind Massageöle beliebig einstellbarer Konsistenz mit Gehalten von hautwirksamen Wirkstoffen wie z. B. Eukalyptusöl oder Auszugsölen von Hyperikum perforatum o. dgl., ölige Behandlungsmittel der Nasenschleimhaut aus insbesondere Paraffinum subliquidum sowie ggf. Gehalten von Eukalyptusöl, Ephedrin oder weiteren Wirkstoffen, Lippenpomaden auf Paraffinbasis sowohl zur Benutzung bei starker Sonneneinstrahlung und dann vorhandenen Gehalten von Lichtschutzmitteln wie auch zur Pflege der Lippen zum Schutz vor Rissigwerden. Zubereitungen für die Zahnheilkunde mit dem Vorteil der guten Haftung an Mundschleimhaut und Zähnen bei Spülen des Mundes mit Wasser sind weiterhin vorgesehen. Flüssige oder pastöse Zubereitungen mit Gehalten von Wirkstoffen oder ohne diese sind zur rektalen Applikation geeignet.

Die weitere Komponente von öliger Konsistenz ist nach dem Gesagten von an sich beliebiger Art, soweit eine pharmazeutische oder kosmetische Unbedenklichkeit und Eignung gegeben sind.

Geeignet sind fette Öle natürlicher und synthetischer Herkunft, die sich von Glyceriden ableitenden synthetisch hergestellten Neutralöle, Paraffinum subliquidum sowie allgemein Ester aus mehrwertigen Alkoholen mit 2 bis 4 Kohlenstoffatomen und gesättigten oder ungesättigten, geradkettigen oder verzweigtkettigen Monocarbonsäuren mit 1 bis 30 Kohlenstoffatomen.

Bevorzugt sind sogenannte Neutralöle, d. h. nicht ranzig werdende Triglyceride der gesättigten geradkettigen Fettsäuren mit 6 bis 14, insbesondere 8 bis 12 Kohlenstoffatomen, sowie entsprechende Triglyceride, in welchen neben den genannten gesättigten Fettsäuren aliphatische Dicarbonsäuren mit 2 bis 6 Kohlenstoffatomen enthalten sind.

Es soll verstanden werden, daß die Komponente von öliger Konsistenz sehr kleine Anteile haben kann oder sogar fortfallen kann, sofern das viskose gelartige Estergemisch nur sehr kleine Anteile Silane als Reaktionskomponente enthält, wobei dann ggf. der Wirkstoff ein pflanzliches wirkstoffhaltiges Preßöl ist.

Die Herstellung der Zubereitungen mit Gehalten von silanmodifizierten Estergemischen kann durch Mischen der Komponenten, gegebenenfalls unter Einfluß von intensiver Rührung oder von Scherwirkung und gegebenenfalls unter Erwärmen erfolgen.

In den Beispielen bedeutet OHZ die Hydroxylzahl SZ die Säurezahl.

Beispiel 1 (viskoses gelartiges Estergemisch)

In einem 1-Liter-Schliffkolben, versehen mit Rührer, Wasserabscheider, Thermometer und Gaseinleitungsrohr, wird eine Mischung aus 150 g Triäthylenglykol, 426 g Isostearinsäure und 0,1 g Tetrabutyltitanat unter Rühren auf 240 °C bei 760 mbar erhitzt, worauf bei gleicher Temperatur innerhalb von 6 Stunden der Druck auf 100 gesenkt wird. Der so gebildete Partialester weist eine Säurezahl unter 1 und eine Hydroxylzahl von 52 auf. Nach Abkühlung auf 100 °C werden 105 g $\gamma$-Glycidyl-oxipropyl-trimethoxisilan und 1 g $AlCl_3$ als Katalysator zugesetzt und verrührt. Bei 180 °C und 100 mbar erfolgt anschließend die Wasserdampfeinleitung bei einer Dampftemperatur von 130 °C durch die vorliegende Reaktionsmischung. Die Dampfdurchleitung wird solange durchgeführt, bis kein weiteres Methanol mehr abgespalten wird. Dabei werden Methanol und Wasser fortwährend als Brüden entfernt. Parallel dazu erfolgt der Viskositätsanstieg des Silan-modifizierten Estergemisches zu einem klaren transparenten Oleogel.

SZ : kleiner 2
Viskosität bei 20 °C = 47 500 mPa · s.
OHZ : 40.

Beispiel 2 (viskoses gelartiges Estergemisch)

In der in Beispiel 1 beschriebenen Reaktionstemperatur wird eine Mischung aus 80 g Propandiol-1,2, 450 g Ölsäure und 0,1 g Tetrabutyltitanat unter den beschriebenen Reaktionsbedingungen zu einem Partialester mit einer Säurezahl von 0,6 und einer Hydroxylzahl von 55 verestert. Bei 80 °C und 1 000 mbar werden 136 g $\gamma$-Glycidyl-oxipropyl-triethoxisilan in Gegenwart von 0,5 g $SnCl_4$ angelagert. Anschließend wird bei 140 °C und 40 mbar Wasserdampf von 120 °C in die Reaktionsmischung eingeleitet. Die Wasserdampfeinleitung erfolgt bis zur vollständigen Ethanolabspaltung. Es wird ein viskoses gelartiges Estergemisch mit streichbarer und transparenter Konsistenz erhalten. Viskosität bei 20 °C = 37 600 mPa · s. OHZ : 39.

Beispiel 3 (viskoses gelartiges Estergemisch)

In einem 1-Liter-Schliffkolben, der mit Rührwerk, Wasserabscheider, Thermometer und Gaseinleitungsrohr versehen ist, werden 500 g eines Triglyceridgemischs aus gesättigter Pflanzenfettsäure mittlerer Kettenlänge von 8 bis 10 C-Atomen und Adipinsäure, wobei 0,55 Mol Adipinsäure und 1,9 Mol genannter Pflanzenfettsäure pro Mol Glycerin verestert wurden sowie 6 g Glycerin und 0,1 g Tetrabutyltitanat unter gleichzeitigem Durchleiten von Inertgas bei 240 °C unter Rühren 4 Stunden erhitzt. Die durch Umesterung erzielte Hydroxylzahl der Reaktionsmischung beträgt 26. Diese Reaktionsmischung, als Bernsteinsäure kondensiertes Fettsäure-partialglycerid vorliegend, wird mit 52 g $\gamma$-Glycidyl-oxypropyltrimethoxysilan und 1 g $AlCl_3$ versetzt und 0,5 Stunden bei 100 °C und 1 000 mbar verrührt. Anschließend wird bei 180 °C und 80 mbar unter Rühren 160 °C heißer Wasserdampf durch den silanmodifizierten Fettsäurepartialester geleitet. Spaltmethanol und Wasserdampf werden als Brüden entfernt. Die Viskosität

des silanmodifizierten Fettsäure-partialesters nimmt mit steigendem Methanolanfall zu, wobei nach Ende der Abspaltung des Methanols ein klares transparentes Gel resultiert.

SZ : 0,7
Verseifungszahl : 394
OHZ : 22
Viskosität bei 20 °C : 10 800 mPas · s
Tropfpunkt höher 300 °C.

Beispiel 4 (viskoses gelartiges Estergemisch)

Entsprechend Beispiel 3 werden 500 g eines Triglycerids aus gesättigten Cocosölfettsäuren mit 8 bis 18 C-Atomen und einer Hydroxylzahl von 9 und 12 g Glycerin in Gegenwart von 0,1 g Tetrabutyltitanat zu einem Fettsäurepartialglycerid mit einer Hydroxylzahl von 49 umgeestert. Die weitere Umsetzung mit 139 g $\gamma$-Glycidyl-oxypropyl-tripropoxysilan und 2 g $SnCl_4$ erfolgt bei 120 °C und 1 000 mbar. Anschließend erfolgt bei 200 °C und 50 mbar Wasserdampfeinleitung mit Dampftemperaturen von 170 °C über einen Reaktionszeitraum von 6 Stunden die Gelierung zu einem hochviskosen Oleogel.

SZ : 0,8
OHZ : 35
Viskosität bei 52 °C : 83 000 Pa · s
Schmelzpunkt : 39,5 °C.

Beispiel 5a (viskoses gelartiges Estergemisch)

Entsprechend Beispiel 1 wird eine Mischung aus 114 g Trimethylolpropan und 555 g Isostearinsäure in Gegenwart von 0,2 g Tetrabutyltitanat zu einem Partialester mit einer Hydroxylzahl von 59 verestert. Die Anlagerung von 156 g $\gamma$-Glycidyl-oxypropyl-trimethoxysilan erfolgt in Gegenwart von 2 g $AlCl_3$ bei 100 °C und 1 000 mbar. Der silanmodifizierte Partialester wird zur Entfernung des Katalysators filtriert und anschließend unter Durchleitung von Wasserdampf mit Dampftemperaturen von 160 bis 180 °C und 80 mbar geliert. Innerhalb von 4 Stunden Reaktionszeit werden Methanol und Wasser als Brüden entfernt.

SZ : 1,4
OHZ : 48
Viskosität bei 20 °C : 82 000 mPas · s.

Beispiel 5b (Verwendung bzw. Zubereitung)

Die Löslichkeit und Viskosität des nach Beispiel 5a hergestellten silanmodifizierten Trimethylolpropan-isostearat-Geles wird in Mischung mit Trimethylolpropantriisostearat untersucht. Die jeweiligen Anteile an viskosem gelartigen Estergemisch und Esterkomponente werden im Becherglas eingewogen und bei 20 bis 25 °C verrührt. Es entstehen insgesamt viskose bis gelartige klare Lösungen bzw. Gel-Systeme.

| Gewichtsprozent Oleogel | Gewichtsprozent Ester | Viskosität bei 30 °C [mPas . s] |
|---|---|---|
| 0 | 100 | 135 |
| 20 | 80 | 840 |
| 40 | 60 | 4380 |
| 50 | 50 | 10500 |
| 60 | 40 | 18000 |
| 80 | 20 | 47500 |
| 100 | 0 | 82000 |

Oleogele mit einer gleichartigen Abstufung der Viskosität werden durch Mischen der Substanzen der Beispiele 1 bis 5 a mit fetten Ölen, vorzugsweise nicht ranzig werdenden Rückveresterungsölen aus Glycerin und gesättigten geradkettigen Monokarbonsäuren der Kettenlänge $C_8$ bis $C_{12}$ sowie mit wirkstoffhaltigen Pflanzenpressölen bzw. Auszugsölen erhalten.

Beispiel 6 (viskoses gelartiges Estergemisch)

Entsprechend Beispiel 1 werden 82 g Pentaerythrit und 613 g Isostearinsäure in Gegenwart von 0,1 g Tetrabutyltitanat zu einem Pentaerythrit-partialester mit einer Hydroxylzahl von 20 verestert. Bei 100 °C erfolgt in Gegenwart von 1 g $AlCl_3$ die Anlagerung von 55 g γ-Glycidyl-oxypropyl-trimethoxysilan. Unter Wasserdampfeinleitung bei Dampftemperaturen von 160 bis 180 °C und 80 mbar kondensiert die Reaktionsmischung innerhalb von 6 Stunden zu einem klaren viskosen gelartigen Estergemisch unter Methanolabspaltung.

OHZ : 14

Viskosität bei 30 °C : 4 800 mPa · s.

Beispiel 7 (viskoses gelartiges Estergemisch)

Entsprechend Beispiel 1 werden 82 g Pentaerythrit und 562 g Isostearinsäure in Gegenwart von 0,1 g Tetrabutyltitanat zu einem Pentaerythrit-partialester mit einer Hydroxylzahl von 40 verestert. Bei 100 °C erfolgt die Anlagerung von 102 g γ-Glycidyl-oxypropyl-trimethoxysilan in Gegenwart von 1 g $AlCl_3$. Unter Wasserdampfeinleitung mit Dampftemperaturen von 160 bis 180 °C und 80 mbar kondensiert die Reaktionsmischung innerhalb von 7 Stunden zu einem klaren viskosen Oleogel unter Methanolabspaltung.

OHZ : 32

Viskosität bei 30 °C : 36 000 mPa · s.

Beispiel 8 (viskoses gelartiges Estergemisch)

Entsprechend Beipiel 1 werden 82 g Pentaerythrit und 511 g Isostearinsäure in Gegenwart von 0,1 g Tetrabutyltitanat zu einem Pentaerythrit-partialester mit einer Hydroxylzahl von 60 verestert. Bei 100 °C erfolgt die Anlagerung von 141 g γ-Glycidyl-oxypropyl-trimethoxysilan in Gegenwart von 1 g $AlCl_3$. Unter Wasserdampfeinleitung mit Dampftemperaturen von 160 bis 180 °C und 80 mbar kondensiert die Reaktionsmischung innerhalb von 8 Stunden zu einem klaren hochviskosen Oleogel unter Methanolabspaltung.

OHZ : 55

Viskosität bei 30 °C : 98 000 Pas · s.

Beispiel 9 (Zubereitung)

In einem Rührkessel werden durch Zusammengeben und Rühren bei 40 °C bis zur homogenen Verteilung der Komponenten das Sonnenschutzgel 1a und das viskose Sonnenschutzöl 1b hergestellt.

| Beispiel | 1a | 1b |
|---|---|---|
| viskoses Estergemisch nach Beispiel 4 | 50 Gew.-Teile | 10 Gew.-Teile |
| Neutralester I XX | 50 Gew.-Teile | 90 Gew.-Teile |
| Neo-Heliopan X | 5 Gew.-Teile | 5 Gew.-Teile |

X Lichtschutzsubstanz der Fa. Haarmann und Reimer, Holzminden

XX Gemischtsäuriges Triglycerid aus 2,1 Mol gesättigten Fettsäuren der Kettenlänge $C_8$ bis $C_{10}$ und 0,45 Mol Bernsteinsäure.

Beide Produkte bewirken ein angenehmes Gefühl auf der Haut. Produkt 1b erzeugt einen sichtbaren, Produkt 1a einen starken Glanz. Die Haut wird nicht benetzbar.

Produkt 1a wird einem Waschtest durch übliches Waschen der Haut mit Seife unterzogen : Noch nach der 3. Wäsche perlt Wasser von der Haut ab ; erst nach der 5. Wäsche ist das Produkt zum größten Teil, nach der 8. Wäsche fast völlig entfernt.

Zum Vergleich wird ein Gel aus dem Neutralester I und einem organisch modifizierten Montmorillonit (Bentone (R), NL-Industries, Inc., Highstown, N.J., USA) in einer zur Erzeugung der gleichen Viskosität ausreichenden Menge verrührt. Dieses Gel wird bereits durch die erste Wäsche mit Seife entfernt.

Beispiel 10 (Zubereitung)

Durch Vermischen von 20 Teilen des Produkts nach Beispiel 5 mit 80 Teilen Neutralöl II (Triglycerid der gesättigten Fettsäuren mittlere Kettenlänge $C_8$ bis $C_{10}$) und 10 Teilen Eukalyptusöl wird ein viskoses Öl zum Einträufeln in die Nase bei Erkältungskrankheiten hergestellt. Weitere Wirkstoffe z. B. Ephedrin können zugegeben werden.

Das Produkt verteilt sich in kleinen Mengen gleichmäßig auf der Nasenschleimhaut. Erst nach mehreren Stunden ist eine erneute Anwendung erforderlich.

Beispiel 11 (Zubereitung)

Durch Vermischen von 20 Teilen des Produkts nach Beispiel 5 mit 80 Teilen Neutralöl II und 5 Teilen einer Mischung aus gleichen Anteilen Melissenöl, Latschenkieferöl und Johanneskantöl (Ol. Hyperici) wird ein Massageöl hergestellt, das sich leicht und gleichmäßig verteilen läßt und bereits nach kurzem Einreiben keine öligen Bestandteile an die Kleidung abgibt.

**Patentansprüche**

1. Viskose gelartige Estergemische bestehend aus Umsetzungsprodukten von Fettsäure-polyol-partialestern oder deren Gemischen mit Hydroxylzahlen von 5 bis 150, vorzugsweise 10 bis 80 und 2 bis 45 Gew.-%, vorzugsweise 4 bis 30 Gew.-%, bezogen auf die Estergemische, von γ-Glycidyl-oxipropyl-trialkoxisilanen mit Alkylgruppen von 1 bis 3-C-Atomen oder deren Gemischen.

2. Estergemische nach Anspruch 1, dadurch gekennzeichnet, daß die Umsetzungsprodukte als weitere Reaktionskomponente Dicarbonsäuren bzw. deren Anhydride bzw. Halogenide in Mengen von 0,01 bis 50 Mol-%, vorzugsweise 0,01 bis 30 Mol-%, je Mol OH-Gruppen der Polyole enthalten.

3. Verfahren zur Herstellung viskoser gelartiger Estergemische nach Anspruch 1, dadurch gekennzeichnet, daß man Fettsäurepolyol-partialester oder deren Gemische mit Hydroxylzahlen von 5 bis 150, bevorzugt 10 bis 80 mit 2 bis 45 Gew.-%, vorzugsweise 4 bis 30 Gew.-%, bezogen auf die Estergemische, von γ-Glycidyl-oxipropyl-trialkoxisilane mit Alkylgruppen von 1 bis 3 C-Atomen bei 20 bis 210 °C, vorzugsweise 60 bis 120 °C, umsetzt und die freien Alkoxigruppen der Silane bei 100 bis 240 °C, vorzugsweise 160 bis 200 °C, unter Wasserdampfdurchleitung bei Dampftemperaturen von 100 bis 240 °C, bevorzugt 150 bis 170 °C, zu viskosen gelartigen Estergemischen ganz oder teilweise siloxanartig kondensiert oder Fettsäure-polyol-partialester mit Hydroxylzahlen von 5 bis 150 durch Umsetzung von Fettsäure-polyol-vollestern durch Umsetzung mit Polyolen bildet und darauf mit 2 bis 45 Gew.-% γ-Glycidyl-oxipropyl-trialkoxysilanen umsetzt und entsprechend siloxanartig kondensiert.

4. Verfahren nach Anspruch 3, dadurch gekennzeichnet, daß man Dicarbonsäuren oder deren Anhydride bzw. Halogenide in Mengen von 0,01 bis 50 Mol.-% OH-Gruppen der Polyole mit Fettsäure-polyol-partialestern zu solchen der Hydroxylzahlen von 5 bis 150 vor der Reaktion mit γ-Glycidyl-oxipropyl-trialkoxysilanen umsetzt.

5. Verwendung der Stoffe nach den Ansprüchen 1 oder 2, dadurch gekennzeichnet, daß man die viskosen gelartiger Estergemische als Gelbildner oder Hilfsstoffe in pharmazeutischen oder kosmetischen Präparaten einsetzt.

6. Streichfähige Zubereitung mit hoher Haftfähigkeit auf der Haut, bestehend aus

a) 5 bis 80 Gew.-% eines gelartigen Estergemisches bestehend aus Umsetzungsprodukten von Fettsäure-polyol-partialestern oder deren Gemischen mit Hydroxylzahlen von 5 bis 150, vorzugsweise 10 bis 80, mit 2 bis 45 Gew.-%, vorzugsweise 4 bis 40 Gew.-%, bezogen auf die Estergemische, von γ-Glycidyl-oxypropyl-trialkoxysilanen mit Alkylgruppen von 1 bis 3 C-Atomen oder deren Gemischen oder gegebenenfalls bestehend aus Umsetzungsprodukten, die als weitere Reaktionskomponente Dicarbonsäuren bzw. deren Anhydride bzw. Halogenide in Mengen von 0,01 bis 50 Mol.-%, vorzugsweise 0,01 bis 30 Mol.-%, je Mol OH-Gruppen der Polyole enthalten (gemäß einem der Patentansprüche 1 oder 2)

b) 95 bis 20 Gew.-% einer Komponente von öliger Konsistenz oder einer W/O- bzw. O/W-Emulsion aus diesem und Wasser sowie gegebenenfalls

c) 0,01 bis 20 Gew.-% eines Wirkstoffs oder dessen flüssiger oder gegebenenfalls fester Zubereitung.

7. Zubereitung nach Anspruch 6 als pharmazeutische oder kosmetische Zubereitung zur Hautbehandlung.

8. Zubereitung nach Anspruch 7 als Hautschutzmittel oder Sonnenschutzmittel bzw. Strahlenschutzmittel.

9. Zubereitung nach Anspruch 7 als Spray-Zubereitung.

**Claims**

1. Viscous gel-type ester mixture consisting of reaction products of fatty acid-polyol partial esters or mixtures thereof with hydroxyl numbers of 5 to 150, preferably 10 to 80 and 2 to 45 % by weight, preferably 4 to 30 % by weight related to the ester mixture of γ-glycidyl-oxypropyl-trialkoxy silanes with alkoxy groups of 1 to 3 C-atoms or mixtures thereof.

2. Ester mixtures according to claim 1, characterised in that the reaction products contain as further reaction components dicarboxylic acids or anhydrides or halides thereof in amounts of 0.01 to 50 mol.-%, preferably 0.01 to 30 mol.-%, per mol OH groups of the polyols.

3. Process for the preparation of viscous gel-type ester mixtures according to claim 1, characterised in that fatty acid polyol partial esters or mixtures thereof with hydroxyl numbers of 5 to 150, preferably 10 to 80 are reacted with 2 to 45 % by weight, preferably 4 to 30 % by weight, related to the ester mixture, of γ-glycidyl-oxypropyl-trialkoxy silanes with alkyl groups of 1 to 3 C-atoms at 20 to 210 °C, preferably 60 to 120 °C and the free alkoxy groups of the silanes are completely or partially condensed in the manner of siloxanes to form viscous gel-type ester mixtures at 100 to 240 °C, preferably 160 to 200 °C, while subject to passage of steam at steam temperatures of 100 to 240 °C, preferably 150 to 170 °C, or fatty acid-polyl partial esters with hydroxyl numbers of 5 to 150 are formed by reaction of fatty acid-polyol complete esters by transesterification with polyols and then reacted with 2 to 45 % by weight of γ-glycidyl-oxypropyl-trialkoxy silanes and correspondingly condensed in the manner of siloxanes.

4. Process according to claim 3, characterised in that dicarboxylic acids or anhydrides or halides thereof are reacted in amounts of 0.01 to 50 mol-% OH groups of the polyols with fatty acid polyol partial esters to form those of hydroxyl numbers of 5 to 150 before the reaction with γ-glycidyl-oxypropyl-trialkoxy silanes.

5. Use of the substances according to claims 1 or 2, characterised in that the viscous gel-type ester mixtures are employed as gel formers or auxiliary substances in pharmaceutical or cosmetic preparations.

6. Spreadable preparation with high adhesion to the skin, consisting of

a) 5 to 80 % by weight of a gel-type ester mixture consisting of reaction products of fatty acid-polyol partial esters or mixtures thereof with hydroxyl numbers of 5 to 150, preferably 10 to 80, with 2 to 45 % by weight, preferably 4 to 30 % by weight, related to the ester mixture, of γ-glycidyl-oxypropyl-trialkoxy silanes with alkyl groups of 1 to 3 C-atoms or mixtures thereof, or optionally consisting of reaction products which contain as further reaction components dicarboxylic acids or anhydrides or halides thereof in amounts of 0.01 to 50 mol.%, preferably 0.01 to 30 mol%, per mol OH-groups of the polyol (according to one of claims 1 or 2)

b) 95 to 20 % by weight of a component of oily consistency or a W/O or O/W emulsion of this and water, as well as optionally

c) 0.01 to 20 % by weight of an active substance or liquid or optionally solid preparation thereof.

7. Preparation according to claim 6 as pharmaceutical or cosmetic preparation for skin treatment.

8. Preparation according to claim 7 as skin screening agent or sun screening agent or radiation screening agent.

9. Preparation according to claim 7 as spray preparation.

**Revendications**

1. Mélange d'esters visqueux de type gel constitué par des produits de réaction d'esters partiels d'acides gras-polyols ou de leurs mélanges possédant des indices d'hydroxyle de 5 à 150, de préférence de 10 à 80 et par de 2 à 45 % en poids, de préférence de 4 à 30 % en poids, par rapport aux mélanges d'esters, de γ-glycidyloxypropyl trialcoxysilanes avec des groupes alkyles de 1 à 3 atomes de C ou leurs mélanges.

2. Mélange d'esters selon la revendication 1, caractérisé en ce que les produits de réaction contiennent, comme autres constituants réactionnels, des acides dicarboxyliques, leurs anhydrides, respect. Leurs halogénures en des quantités allant de 0,01 à 50 % molaire, de préférence de 0,01 à 30 % molaire, par mole de groupes OH des polyols.

3. Procédé de préparation de mélanges d'esters visqueux de type gel selon la revendication 1, caractérisé en ce que l'on fait réagir des esters partiels d'acides gras-polyols ou leurs mélanges possédant des indices d'hydroxyle de 5 à 150, de préférence de 10 à 80, avec 2 à 45 % en poids, de préférence 4 à 30 % en poids, par rapport aux mélanges d'esters, de γ-glycidyloxypropyl trialcoxysilanes avec des groupes alkyles de 1 à 3 atomes de C, entre 20 et 210 °C, de préférence entre 60 et 120 °C et que l'on condense les groupes alcoxy libres des silanes, entièrement ou partiellement à la manière des siloxanes, à 100 jusqu'à 240 °C, de préférence de 160 à 200 °C, avec barbotage de vapeur d'eau à des températures de la vapeur de 100 à 240 °C, de préférence de 150 à 170 °C, en des mélanges d'esters visqueux de type gel ou que l'on forme des esters partiels d'acides gras-polyols avec des indices d'hydroxyle de 5 à 150 par réaction d'esters totaux d'acides gras-polyols avec des polyols, que l'on fait réagir ensuite avec 2 à 45 % en poids de γ-glycidyloxypropyl trialcoxysilanes et condense à la manière des siloxanes.

4. Procédé selon la revendication 3, caractérisé en ce que, avant la réaction avec des γ-glycidyloxypropyl trialcoxysilanes, on fait réagir des acides dicarboxyliques ou leurs anhydrides, respectivement leurs halogénures en quantités de 0,01 à 50 % molaire de groupes OH des polyols avec des esters partiels d'acides gras-polyols en esters ayant des indices d'hydroxyle de 5 à 150.

5. Utilisation des produits selon la revendication 1 ou 2, caractérisée en ce que l'on met en œuvre les mélanges d'esters visqueux de type gel en tant que gélifiants ou adjuvants dans des préparations pharmaceutiques ou cosmétiques.

6. Préparation facile à étaler présentant une forte adhérence à la peau, constituée par

a) 5 à 80 % en poids d'un mélange d'esters de type gel consistant en des produits de réaction d'esters partiels d'acides gras-polyols ou de leurs mélanges possédant des indices d'hydroxyle de 5 à 150,

de préférence de 10 à 80, avec 2 à 45 % en poids, de préférence 4 à 30 % en poids, par rapport aux mélanges d'esters, de γ-glycidyloxypropyl trialcoxylsilanes avec des groupes alkyles de 1 à 3 atomes de C ou de leurs mélanges ou consistant, le cas échéant, en mélanges d'esters qui contiennent, comme autres constituants réactionnels, des acides dicarboxyliques, respectivement leurs anhydrides ou des halogénures en quantités allant de 0,01 à 50 % molaire, de préférence de 0,01 à 30 % molaire, par mole de groupes OH des polyols (selon l'une des revendications 1 ou 2),

b) 95 à 20 % en poids d'un constituant de consistance huileuse ou d'une émulsion aqueuse ou huileuse à partir de ce constituant et d'eau, ainsi qu'éventuellement

c) 0,01 à 20 % en poids d'un constituant actif ou de sa préparation fluide ou éventuellement solide.

7. Préparation selon la revendication 6 en tant que préparation pharmaceutique ou cosmétique pour le traitement de la peau.

8. Préparation selon la revendication 7 en tant qu'agent protecteur de la peau ou agent antisolaire, respectivement agent de protection contre les radiations.

9. Préparation selon la revendication 7 en tant que spray.